# EUROPEAN PATENT APPLICATION

(11) **EP 4 835 071 A1**
(43) Date of publication of application: **04.11.2026**
(21) Application number: 25173300.2
(22) Date of filing: 29.04.2025
(51) Int. Cl.: C12N 15/10

(54) **A METHOD FOR IDENTIFYING PAIRED IMMUNE RECEPTOR CHAIN SEQUENCES**

(71) Applicant: ImmuneDiscover Sweden AB, 104 51 Stockholm (SE)
(72) Inventor: KARLSSON HEDESTAM, Gunilla, 115 20 STOCKHOLM (SE); CORCORAN, Martin, 112 55 STOCKHOLM (SE)
(74) Representative: Kransell & Wennborg KB

(57) **Abstract**

The present disclosure generally relates to a method for identifying paired immune receptor chain sequences. The present disclosure also relates to a method for generating monoclonal antibodies or T-cell receptors.

## Description

### TECHNICAL FIELD

The present disclosure generally relates to a method for identifying paired immune receptor chain sequences. The present disclosure also relates to a method for generating monoclonal antibodies or T-cell receptors.

### BACKGROUND

The immune system is a network of biological processes that protects organisms from diseases. A key component of the immune system is the adaptive immune system, which may generate highly specific responses against pathogens, and may establish immunological memory. This ability enables the adaptive immune system to respond rapidly and specifically to previously encountered antigens, forming the foundation of immunization strategies.

B-cell receptors (BCRs) and T-cell receptors (TCRs) are important components of the adaptive immune system. BCRs and TCRs enable recognition and response to a vast number of antigens.

BCRs are membrane-bound immunoglobulins composed of two identical heavy chains and two identical light chains. These chains are encoded by recombined variable (V), diversity (D), and joining (J) gene segments, which together create the antigen-binding site.

TCRs are heterodimeric proteins, which are typically composed of one alpha (α) chain and one beta (β) chain (or gamma (γ) and delta (δ) chains in less common TCR subsets). As with BCR chains, the TCR chains are formed through somatic recombination of V(D)J gene segments, resulting in antigen-specific receptors.

The pairing of receptor chains, such as heavy and light chains in BCRs or alpha and beta chains in TCRs, is critical for antigen specificity and receptor function.

The theoretical diversity of immune receptor repertoires is immense, estimated to encompass between 10¹⁰ and 10¹⁴ unique specificities. This diversity arises from the recombination of multiple gene segments. For BCRs, there are approximately 152 V, D, and J gene segments, while TCRs utilize around 193 segments, resulting in a total of 345 segments across both receptor types. Each segment may exist in multiple allelic variants, and individuals may also exhibit deletions or duplications of certain segments, further increasing genetic diversity (and complexity). This variation is compounded by differences in homozygosity and heterozygosity across individuals, making the characterization of germline immune receptor genes a complex challenge.

Despite the availability of next-generation sequencing (NGS) technologies, studying BCR and TCR repertoires is a challenge, and is limited by the complexity of the loci encoding these receptors. Current methods often fail to resolve paired receptor chains accurately or efficiently, which is a significant barrier to understanding immune receptor diversity and antigen-specific responses.

The ability to identify and analyze paired receptor chains is important for research and therapeutic applications, including the generation of monoclonal antibodies and engineered T-cell therapies. However, existing methods often struggle with accurate chain pairing, scalability, and sensitivity, particularly when analyzing large immune cell populations or isolating antigen-specific cells.

Furthermore, transitioning from sequencing data to functional expression of immune receptors remains inefficient and prone to error, hindering the development of reliable therapeutic candidates.

In view of this, there is a need for improved methodologies to identify, analyze, and functionally express paired immune receptor chains with precision, scalability, and efficiency.

### SUMMARY

In view of the above-mentioned problems, it is an object of the present disclosure to provide a high-throughput solution for the identification, analysis, and expression of paired immune receptor chains.

According to a first aspect of the present disclosure, there is provided method for identifying paired immune receptor chain sequences comprising:
a) providing a plurality of individual cDNA templates, wherein the individual cDNA templates are synthesized from RNA extracted from single immune cells; the immune cells being B-cells or T-cells;
b) performing polymerase chain reactions (PCRs) on the individual cDNA templates to separately amplify immune receptor chain sequences selected from:
   i) immunoglobulin heavy chain (IGH), kappa light chain (IGK), and lambda light chain (IGL) if the immune cells are B-cells; and
   ii) T-cell receptor alpha chain (TCRα), T-cell receptor beta chain (TCRβ), T-cell receptor gamma chain (TCRγ), and T-cell receptor delta chain (TCRδ) if said immune cells are T-cells, wherein

   step b) comprises subjecting the individual cDNA templates to a set of target primers targeting the immune receptor chain sequences, wherein the set of target primers comprises a plurality of reverse target primers and forward target primers; each of the reverse target primers comprising a reverse bridging sequence, and each of the forward target primers comprising a forward bridging sequence, wherein
   step b) is performed in separate wells of at least one first multiwell plate, and wherein an amplified library is obtained in each well;
c) combining the amplified libraries associated with the same individual cDNA template such that a set of combined libraries is provided;
d) labelling each combined library in the set of combined libraries by performing individual labelling amplification reactions in separate wells of a plurality of second multiwell plates by contacting each combined library with a well-index primer pair, and a plate-index primer pair;
   the well-index primer pair, comprising a reverse well-index primer and a forward well-index primer;
      the reverse well-index primer comprising a first reverse well-bridging sequence targeting the reverse bridging sequence, a second reverse well-bridging sequence, and a reverse well-specific index sequence arranged between the first and second reverse well-bridging sequences;
      the forward well-index primer comprising a first forward well-bridging sequence targeting the forward bridging sequence, a second forward well-bridging sequence, and a forward well-specific index sequence arranged between the first and second forward well-bridging sequences, wherein
   the plate-index primer pair comprises a reverse plate-index primer and a forward plate-index primer;
      the reverse plate-index primer comprising at least a first reverse plate-bridging sequence targeting the second reverse well-bridging sequence;
      the forward plate-index primer comprising at least a first forward plate-bridging sequence targeting the second forward well-bridging sequence, wherein at least one of the reverse and forward plate-index primers comprises a plate-specific index sequence arranged 5' of the first reverse plate-bridging sequence or the first forward plate-bridging sequence, wherein
   different well-index primer pairs are used for each well within the plurality of second multiwell plates, and wherein identical plate-index primer pair is used for each well within each individual second multiwell plate of the plurality of second multiwell plates,
   whereby a labelled amplified library is obtained in each well of the plurality of second multiwell-plates;
e) combining the labelled amplified libraries of each well of the plurality of second multiwell-plates to yield a combined labelled library;
f) sequencing the combined labelled library to provide a sequenced library;
g) analyzing the sequenced library to identify the pairing of immune receptor chain sequences derived from each individual cDNA template.

The present disclosure is based on the realization that a method as defined hereinabove may effectively identify and analyze paired immune receptor chain sequences with high precision in a high throughput manner. With the method of the present disclosure, immune receptor chain sequences, i.e. BCR and TCR chain sequences derived from the same immune cell (either B-cells or T-cells) may be paired. This is important for understanding antigen specificity, immune diversity, and functional immune responses.

In the first step (step a), a plurality of individual cDNA templates is provided. Each cDNA template is extracted from RNA derived and isolated from a single B-cell or T-cell. An individual cDNA template represents the complete repertoire of receptor chain sequences derived from a single B-cell or T-cell.

Individual cDNA templates are provided in separate wells of one or a plurality of first multiwell plates. Hence, each well contains a unique cDNA template.

The method ensures that receptor chains from the same single immune cell are uniquely identifiable, preserving the natural pairing of heavy and light chains in BCRs, or e.g. alpha and beta chains in TCRs.

In step b) a set of target primers designed to amplify the immune receptor chain sequences (either the BCR chain sequences or the TCR chain sequences) is utilized.

The well-index and plate-index primers introduced in step d) introduce unique identifiers or "labels" (well-index sequences and at least one plate-index sequence), which allow for precise tracking of receptor chain sequences in subsequent steps. These indices ensure that the sequences can be traced back to their respective wells and plates; i.e. to the original single B-cell or T-cell.

The labelled amplified products, referred to as labelled amplified libraries, obtained in step d) are subsequently combined (step e), and thereafter sequenced to generate a sequenced library encompassing all of the BCR or TCR chain sequences (step f). This sequenced library is subsequently analyzed to identify paired BCR heavy and light chain sequences or paired TCR alpha and beta chain sequences (or paired TCR gamma and delta chain sequences).

The well-specific and plate-specific index sequences introduced in step d) enable precise "demultiplexing" of receptor sequences after sequencing, which allows for accurate pairing of immune receptor chains.

The accurately paired receptor chain sequences identified by this method can be used for therapeutic applications, such as the generation of monoclonal antibodies or engineered TCRs, which may be used in immunotherapy.

A significant advantage of the present disclosure is that the method enables high throughput and simultaneous processing of large numbers of samples across large populations, making it valuable in both research, diagnostic, and clinical applications, including personalized immunotherapy.

Each of the immune receptor chain sequences comprises
- a variable (V) region, a joining (J) region, and optionally a diversity (D) region;
- a leader region forming the 5' end of each of the immune receptor chain sequences; and
- a constant (C) region forming the 3' end of each of the immune receptor chain sequences.

Each immune receptor chain sequence consists of structurally distinct regions that determine its antigen specificity, expression, and function. The V(D)J regions undergo somatic recombination in B-cells and T-cells, forming unique antigen-binding sites for receptors. This process generates the vast diversity necessary for the (adaptive) immune system to recognize a broad range of antigens.

The heavy chain (IGH) of B-cell receptor chains (BCRs) contains V, D, J, and C regions.

The light chains (IGK, IGL) of BCRs contain V, J, and C regions.

The alpha (TCRα) and gamma (TCRγ) chains of T-cell receptor chains (TCRs) contain V, J, and C regions.

The beta (TCRβ) and delta (TCRδ) chains of TCRs contain V, D, J, and C regions.

The leader region (5' end) is a short sequence required for proper receptor expression. It is present in both BCR and TCR chains and ensures correct processing during transcription and translation.

The constant (C) region (3' end) encode the effector regions of the B-cell and T-cell receptors (e.g., the Fc region in antibodies), contributing to structural stability and function of the receptor.

Amplifying and sequencing of the V, D, J, and C regions is advantageous for reconstructing full immune receptor sequences, as they provide critical information about the specificity and functionality of the receptors.

Typically, the reverse target primer targets the constant region of the immune receptor chain sequence, and wherein the forward target primer targets the leader region of the immune receptor chain sequence.

The reverse target primer typically binds near the 5' end of the constant (C) region.

By targeting these specific regions, the method ensures accurate amplification and sequencing of complete immune receptor chain sequences.

In exemplary embodiments, the forward target primer may comprise the forward bridging sequence and a forward target sequence; the forward target sequence targeting the leader region, wherein the forward bridging sequence is arranged 5' of the forward target sequence, and wherein the reverse target primer comprises
- the reverse bridging sequence, and wherein the reverse bridging sequence targets the constant region; or
- the reverse bridging sequence and a reverse target sequence; the reverse target sequence targeting the constant region, wherein the reverse bridging sequence is arranged 5' of the reverse target sequence.

The forward target sequence may be configured to hybridize to the leader region of the immune receptor chain sequence. The forward bridging sequence may be utilized as a bridge for subsequent amplifications (in the labelling step d)).

In some embodiments, the reverse target primer contains only the reverse bridging sequence. In such embodiments, the reverse bridging sequence is both configured to hybridize to the constant region and to simultaneously act as a bridge for subsequent amplifications.

However, it is equally conceivable that the reverse target primer has the same general construction as the forward target primer. Hence it may comprise a reverse bridging sequence (acting only as a bridge for subsequent amplifications) and a reverse target sequence being configured to target the constant region.

The sequencing in step f) is typically performed using next-generation sequencing (NGS).

NGS enables simultaneous sequencing of large numbers of labelled amplified products. This way, a vast diversity of immune receptor chain sequences in a sample may be analyzed. Accordingly, the method is suitable for applications involving large populations of immune cells. The data output from NGS can be processed using e.g. bioinformatics tools to identify paired receptor chains, and to provide a detailed characterization of the immune receptor repertoire. This enables downstream analyses, e.g. antigen specificity and receptor clonotype/functionality.

In exemplary embodiments, the analyzing in step g) may comprises evaluating the variable (V), joining (J), diversity (D), and/or constant (C) regions associated with the immune receptor chain sequences to identify receptor clonotype composition, and/or assess sequence diversity

Hence, in this step, the V, D, J and C gene usage of each immune receptor chain may be identified. Accordingly, the prevalence and distribution of specific gene segments in the sequenced library may be determined. This way, the receptor clonotype composition may be determined. A receptor clonotype is defined by unique combinations of V(D)J recombinations and CDR3 sequences, which can provide valuable insight to antigen binding and specificity. Furthermore, evaluating the sequence diversity, i.e. the variety within the repertoire of immune receptor chains may also reveal valuable insights on antigen binding.

In exemplary embodiments, step g) comprises a bioinformatic analysis of the sequenced library generated in step f).

In exemplary embodiments, step g) comprises
i) extracting, from the sequenced library, the combined amplified libraries belonging to a specific second multi-well plate by using the plate-specific index sequence(s);
ii) extracting, from the sequenced library, the combined amplified libraries belonging to a specific well by using the well-specific index sequences;
iii) evaluating the variable (V), joining (J), diversity (D), and/or constant (C) regions associated with each combined amplified library;
iv) comparing the sequences of the variable (V), joining (J), diversity (D), and/or constant (C) regions in each combined amplified library with a reference database to evaluate the gene segment usage, identify allelic variants and/or to assign lineage specific complementary determining region 3 (CDR3) sequences for each combined amplified library; and optionally
v) determining a consensus immunoglobulin heavy chain (IGH) and a consensus light chain, selected from kappa light chain (IGK) and lambda light chain (IGL) for the B-cell receptor chains, and/or a consensus T-cell receptor alpha or gamma chain, and a consensus T-cell receptor beta or TCR delta chain for the T-cell receptor chains

The bioinformatic workflow may first demultiplex the sequence data by using the well-specific and the plate-specific indexes to associate sequences with their corresponding single cells.

Subsequently, immune receptor chain sequences may be assigned to their respective gene segments by aligning them to the reference database. This enables the identification of the V, D, and J gene segments used in each chain, as well as the determination of receptor clonotypes and sequence diversity.

This approach provides insights into the diversity and functional repertoire of adaptive immune receptors in a high-throughput manner.

In exemplary embodiments, step g) is performed by a processing unit.

In exemplary embodiments, the reverse and forward target primers of the set of target primers are inner target primers; the set of target primers further comprising a plurality of outer target primers, wherein the polymerase chain reactions (PCRs) in step b) are nested PCR amplifications comprising:
- a first PCR amplification, in which the plurality of outer target primers is used to generate a plurality of amplicons; and
- at least a second PCR amplification, in which the inner target primers are used to anneal sequences within the amplicons generated in the first PCR amplification.

Accordingly, step b) involves performing sequential PCR reactions with two distinct sets of primers: outer target primers for the first PCR and inner target primers for the second PCR. The outer target primers amplify a broader region, while the inner target primers are designed to bind specifically within the amplicons generated by the first PCR.

This approach may be beneficial to enhance the specificity of the amplification process, e.g. by reducing the likelihood of non-specific binding or amplification of unintended regions. The nested design ensures that only the desired target sequences are amplified in the second PCR. Furthermore, this approach may improve the sensitivity, and may allow successful amplification of low-abundance targets that would otherwise remain undetected. This is particularly beneficial when working with complex or challenging samples, where template availability may be limited.

In exemplary embodiments, the ratio of the well-index primer pairs to the plate-index primer pairs used in the method may be at least 1:10, preferably from 1:20 to 1:60.

The well-index primers are rate-limiting and are typically used in lower concentrations. The plate-index primers bind to amplification product of the well index primer reaction. The plate-index primers are typically used in higher concentrations.

In exemplary embodiments, the single immune cells are single B-cells, and wherein the set of target primers is configured to target:
- the IGHV genes, and comprises the forward target primers defined by SEQ ID NO:3-37 and/or SEQ ID NO:44-69 or corresponding sequences having at least 95 %, preferably at least 99% sequence homology with SEQ ID NO:3-37 and/or SEQ ID NO:44-69, and at least one of the reverse target primers defined by SEQ ID NO:38-43, and/or SEQ ID NO:70 or corresponding sequences having at least 95 %, preferably at least 99% sequence homology with SEQ ID NO:38-43, and/or SEQ ID NO:70, and/or
- the IGK genes, and comprises the forward target primers defined by SEQ ID NO:71-86, and/or SEQ ID NO:88-108, or corresponding sequences having at least 95%, preferably at least 99 % sequence homology with SEQ ID NO:71-86, and/or SEQ ID NO:88-108, and the reverse target primers defined by SEQ ID NO:87, and/or SEQ ID NO:109, or corresponding sequences having at least 95%, preferably at least 99 % sequence homology with SEQ ID NO:87, and/or SEQ ID NO:109, and/or
- the IGL genes, and comprises the forward target primers defined by SEQ ID NO:110-141, and/or SEQ ID NO:143-175 or corresponding sequences having at least 95%, preferably at least 99 % sequence homology with SEQ ID NO:110-141, and/or SEQ ID NO:143-175, and the reverse target primers defined by SEQ ID NO:142, and/or SEQ ID NO:176 or corresponding sequences having at least 95%, preferably at least 99 % sequence homology with SEQ ID NO:142 and/or SEQ ID NO:176.

In exemplary embodiments, the method may further comprise a step of performing a retailing polymerase chain reaction (PCR) after step f), wherein the retailing PCR utilizes a set of retailing primers configured to amplify and extend the leader and the constant sequences.

The leader sequence and the constant sequence are intrinsic to each immune receptor chain. The purpose of the retailing PCR step is to extend these sequences, and to introduce new sequence element (e.g. overlaps) to facilitate directional cloning into an expression vector.

The leader sequence is naturally found upstream of the variable (V) region and is responsible for guiding the receptor to the secretory pathway. The constant region, on the other hand, is downstream of the joining (J) region and contributes to effector functions.

According to another aspect, there is provided a method for generating monoclonal antibodies or T-cell receptors comprising:
i) identifying paired adaptive immune receptor chain sequences as described hereinbefore;
ii) cloning at least one or a plurality of paired adaptive immune receptor chain sequences identified in step i) into an expression vector; and
iii) expressing the paired immune receptor chain sequences in a host cell to produce monoclonal antibodies or T-cell receptors.

The method may be used to study immune receptor diversity and function and represents a valuable tool for immunological research and drug discovery. By preserving the natural pairing of immune receptor chains throughout cloning and expression, antigen-binding properties and functional characteristics of immune receptors may be determined with high fidelity. This is beneficial for antibody screening and optimization, and TCR functionality studies to advance the development of TCR-based therapies. Furthermore, the method enables detailed studies of immune receptor repertoires, which may provide insights into immune responses to infections, cancer, and autoimmunity.

The method may also be used in personalized vaccine and treatment strategies, including precision immunotherapy, personalized vaccines, and biomarker discovery. For example, the monoclonal antibodies may be tailored to recognize patient-specific tumor antigens or pathogenic epitopes enabling targeted treatments. Also, engineered TCRs may be customized for adoptive T-cell therapies, enhancing their efficacy in targeting tumor or viral antigens unique to a patient's immune profile.

Furthermore, by studying patient-specific immune receptor repertoires, personalized vaccines may be developed. Moreover, monoclonal antibodies and TCRs generated using the method can be employed in diagnostics, e.g. to detect disease-specific biomarkers.

The paired immune receptor chains are preserved throughout the steps of the method, thereby maintaining functional specificity of the immune receptors, and reducing inefficiencies and errors.

In exemplary embodiments, the expression vector in step ii) is a plasmid configured for expression in mammalian cells.

Mammalian expression systems closely mimic the natural environment of immune receptor production, ensuring proper folding, glycosylation, and post-translational modifications that are critical for receptor functionality and stability.

In exemplary embodiments, the cloning in the step (ii) comprises joining the immune receptor chain sequences and the expression vector by a ligase-free process.

Ligase-free cloning, such as Gibson assembly uses homologous recombination to join DNA fragments without the need for restriction enzymes or ligases. This type of cloning allows for a precise and customized assembly of DNA sequences, and multiple fragments may be joined in a single reaction. This way, the time and complexity associated with traditional ligation-based cloning is reduced.

The retailing PCR, as described hereinbefore, may prepare the immune receptor chain sequences for efficient incorporation into an expression vector by adding specific 5' and 3' extensions to the sequences. These extensions provide homology to the expression vector and facilitate the integration during the cloning process.

The cloning step (step ii) may utilize these extensions to join the retailing PCR products with the expression vector in a ligase-free process, such as Gibson assembly. This process aligns complementary regions on the PCR products and vector, enabling homologous recombination without the need for enzymatic ligation.

Hence, the cloning in the step (ii) may comprise joining the retailing PCR products obtained in the retailing PCR step and the expression vector by a ligase-free process.

However, the method is by no means limited to this specific cloning procedure.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Figure 1 schematically illustrates the steps of the method according to an exemplary embodiment of the present disclosure.
Figure 2A schematically illustrates the library amplification step (step b) of the method according to an exemplary embodiment of the method of the present disclosure.
Figure 2B schematically illustrates the library amplification step (step b) of the method according to an alternative exemplary embodiment of the method of the present disclosure.
Figure 3A schematically illustrates the well and plate indexing step (step d) of the method according to an exemplary embodiment of the present disclosure and being connected to the amplification step of figure 2A.
Figure 3B schematically illustrates the well and plate indexing step (step d) of the method according to an alternative exemplary embodiment of the present disclosure and being connected to the amplification step of figure 2B.

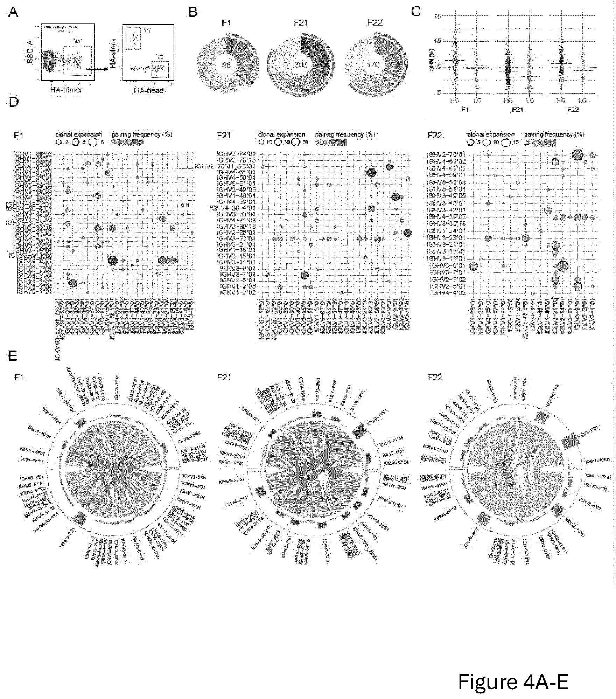

Figure 4 reflects the results in the Example section when studying volunteers with a history in Influenza A exposure. (A) Representative gating strategy identifying HA trimer/HA head or HA trimer/HA stem-binding cells from CD19⁺CD20⁺ IgG⁺IgD⁻IgM⁻ memory B cells for isolation of paired HC and LC sequences. (B) Donut plots summarizing the total number of paired sequences from HA-trimer binding cells analyzed for each donor (F1: n=96; F21: n=397, F22: n=172). Colored pie slices and grey bars indicate expanded clonotypes, proportionally sized, isolated from the same individual. Clonotypes clustering was based on the paired chain algorithm of the IgDiscover clonotypes module (single-linkage clustering based on identical V and J allele assignments and CDR3 length with 80% HCDR3 sequence homology). (C) Linked IGHV and IGKV/IGLV allele usage of HA-trimer binding memory B cells in study participants F1, F21 and F22. Relative frequencies of clonally collapsed V allele pairings encoded by color transparency. (D) Chord diagrams representing HC and LC V allele pairings linked to bulk IgM and IgK/L repertoire frequencies. Alleles with the same amino acid sequence present in a donor were combined. Sectors reflect the usage prevalence of single alleles and connecting arcs are proportional to the frequency of co-occurrence of each combination within the HA trimer-specific response. Bar heights and orientation show the gene usage frequencies of the HA response in relation to that of the total IgM and IgK/L repertoires.

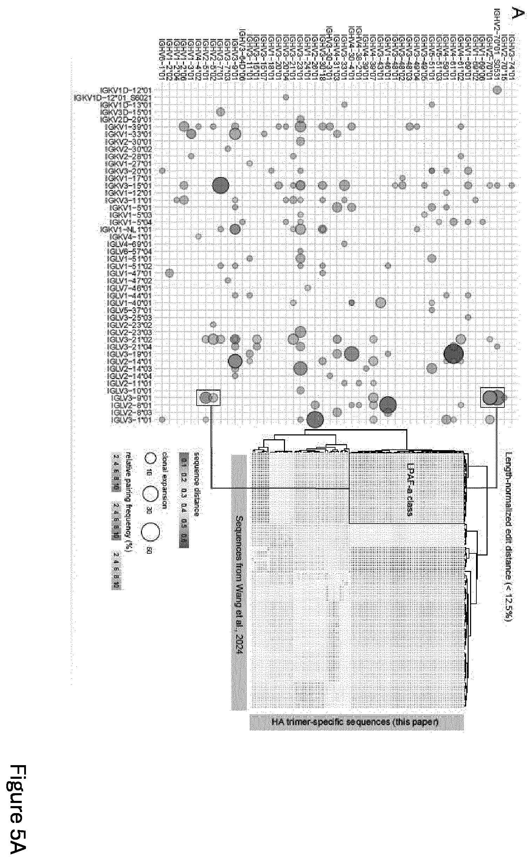

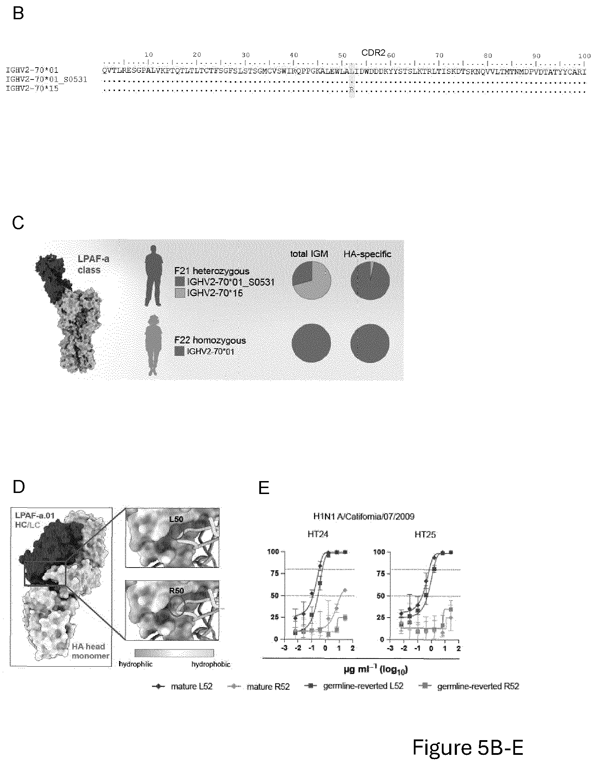

Figure 5 reflects the results in the Example section when studying the biased germline allele usage in a multi-donor class of HA head-targeting antibodies. (A) Cross-donor comparison of IGH and IGK/L V pairing distribution. Length-normalized edit distance between a recently published dataset [4] and all 659 HA trimer-specific sequences obtained in this study were calculated in pairwise string alignment. HC nucleotide sequence dissimilarity of known and novel HA-targeted mAbs were displayed as a heatmap (< 12.5% nucleotide heterology) with clusters of least distance comprising the LPAF-a class. **(B)** Aligned amino acid sequences of the IGHV2-70 alleles present in F21 and F22 with the coding difference in amino acid position 50 (Kabat) highlighted in grey. (C) Left: Structure of LPAF-a.01 (PDB 6URM, dark grey) and H1 CA09 HA (PDB 4M4Y, light grey) illustrates the Ab target. Right: Usage preference of IGHV2-70*01_S0531 over IGHV2-70*15 is HA trimer-specific in F21 when compared to expressed IgM bulk data. (D) Structural prediction of IGHV2-70 L50R. Left: surface of PDB 6URM with HA in light grey, LPAF-a.01 HC in dark grey, LPAF-a.01 LC in grey, and HC position 50 marked with an asterisk. Right: insets of LPAF-a.01 HC in hydrophobicity surface rendering and LC in grey with HC L50 (top) or R50 (bottom, manually mutated to arginine in ChimeraX). (E) Virus neutralizing activity of two LPAF-a.01 class antibodies, HT24 and HT25, in their mature and germline-reverted form showing that an R in position 50 reduces or abolishes their activity.
Figure 6 schematically illustrates the computational workflow according to an exemplary embodiment of the method of the present disclosure.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

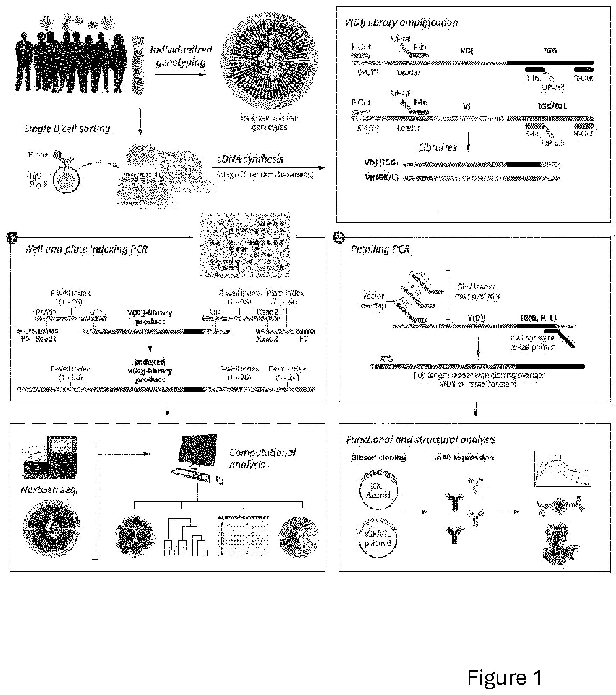

Figure 1 schematically outlines a method for identifying paired immune receptor chain sequences according to an exemplary embodiment of the present disclosure. The method comprises:
a) providing a plurality of individual cDNA templates, wherein the individual cDNA templates are synthesized from RNA extracted from single immune cells; the immune cells being B-cells or T-cells;
b) performing polymerase chain reactions (PCRs) on the individual cDNA templates to separately amplify immune receptor chain sequences 1 selected from:
   i) immunoglobulin heavy chain (IGH), kappa light chain (IGK), and lambda light chain (IGL) if the immune cells are B-cells; and
   ii) T-cell receptor alpha chain (TCRα), T-cell receptor beta chain (TCRβ), T-cell receptor gamma chain (TCRγ), and T-cell receptor delta chain (TCRδ) if the immune cells are T-cells, wherein

   step b) comprises subjecting the individual cDNA templates to a set of target primers targeting the immune receptor chain sequences 1, wherein the set of target primers comprises a plurality of reverse target primers 2 and forward target primers 2'; each of the reverse target primers 2 comprising a reverse bridging sequence 4, and each of the forward target primers 2' comprising a forward bridging sequence 4', wherein
   step b) is performed in separate wells of at least one first multiwell plate, and wherein an amplified library 5 is obtained in each well;
c) combining the amplified libraries 5 associated with the same individual cDNA template such that a set of combined libraries 5' is provided;
d) labelling each combined library 5' in the set of combined libraries by performing individual labelling amplification reactions in separate wells of a plurality of second multiwell plates by contacting each combined library 5' with a well-index primer pair (6, 6') and a plate-index primer pair 7, 7';
   the well-index primer pair (6, 6') comprising a reverse well-index primer 6 and a forward well-index primer 6';
   the reverse well-index primer 6 comprising a first reverse well-bridging sequence 8 targeting the reverse bridging sequence 4, a second reverse well-bridging sequence 9, and a reverse well-specific index sequence 10 arranged between the first 8 and second 9 reverse well-bridging sequences;
   the forward well-index primer 6' comprising a first forward well-bridging sequence 8' targeting the forward bridging sequence 4', a second forward well-bridging sequence 9', and a forward well-specific index sequence 10' arranged between the first 8' and second 9' forward well-bridging sequences, wherein
   the plate-index primer pair (7,7') comprises a reverse plate-index primer 7 and a forward plate-index primer 7';
   the reverse plate-index primer 7 comprising at least a first reverse plate-bridging sequence 11 targeting the second reverse well-bridging sequence 9;
   the forward plate-index primer 7' comprising at least a first forward plate-bridging sequence 11' targeting the second forward well-bridging sequence 9', wherein at least one of the reverse 7 and forward 7' plate-index primers comprises a plate-specific index sequence (12,12') arranged 5' of the first reverse plate-bridging sequence 11 or the first forward plate-bridging sequence 11', wherein
   different well-index primer pairs (6, 6') are used for each well within the plurality of second multiwell plates, and wherein identical plate-index primer pairs (7,7') are used for each well within each individual second multiwell plate of the plurality of second multiwell plates,
   whereby a labelled amplified library 13 is obtained in each well of the plurality of second multiwell-plates;
e) combining the labelled amplified libraries 13 of each well of the plurality of second multiwell-plates to yield a combined labelled library;
f) sequencing the combined labelled library to provide a sequenced library;
g) analyzing the sequenced library to identify the pairing of immune receptor chain sequences derived from each individual cDNA template.

As used herein, the term "cDNA template" refers to a complementary DNA (cDNA) molecule synthesized from mRNA through the process of reverse transcription. The cDNA template retains the coding sequence of the original RNA molecule, excluding introns and non-coding regions present in genomic DNA, but retaining the 5' and 3' untranslated regions of the mRNA.

In the context of the present disclosure, an "individual cDNA template" represents immune receptor chain sequences derived from a single immune cell, i.e. B-cell or T-cell. The cDNA template serves as the starting material for amplification, sequencing, and analysis of paired immune receptor chain sequences.

Step a) of providing a plurality of cDNA templates may comprise:
- isolating single immune cells from a biological sample;
- labelling the single immune cells with an antigen-specific probe,
- sorting antigen-specific single immune cells into individual wells of the at least one multiwell plate, preferably by flow cytometry;
- lysing the antigen-specific single immune cells to release RNA; and
- synthesizing cDNA from the RNA of the lysed immune cells.

By isolating single immune cells (B-cells or T-cells) from a biological sample, the pairing of receptor chains unique to each single cell is preserved. This single-cell specificity allows for accurate downstream analysis of paired receptor chain sequences.

Labelling the immune cells with an antigen-specific probe allows for the provision of immune cells engaged in recognizing a specific antigen.

Sorting the labelled antigen-specific cells into individual wells of a multiwell plate, preferably by flow cytometry, ensures that each well contains the genetic material from a single immune cell.

Lysis of the immune cells releases RNA, which serves as the template for synthesizing cDNA. The use of RNA as a starting material ensures that the synthesized cDNA reflects the expressed receptor chain sequences of the immune cell at the time of sampling.

For example, individual cells may be sorted into a single well of a multiwell plate (e.g. a PCR plate comprising 96 wells) using an antigen specific probe, and by flow cytometry. Each well may contain a lysis solution, e.g. 4 µl of lysis buffer. The lysis buffer may contain 10x PBS, 100mM DTT plus 40 U/µl RNAase inhibitor.

Subsequently, the plates may be frozen, e.g. at -80°C after sorting. The plates are thawed prior to cDNA synthesis.

A cDNA template for single cell PCR amplification of immune receptor chains (e.g. antibody heavy and light chains) is then transcribed from the RNA released from the lysed cells. This may be accomplished by using a mixture of random hexamer and oligo dT primers with the addition of dNTPs and a reverse transcriptase, e.g. Superscript IV.

After the cDNA templates have been provided, separate amplification reactions are performed to amplify the B-cell receptor chain sequences IGH, IGK, and IGL, and/or the T-cell receptor chain sequences TCRα, TCRβ, TCRγ, and TCRδ (step b).

The separate amplification reactions are typically carried out on separate first multiwell plates.

For example, if the B-cell receptors are to be evaluated, the cDNA templates of step a) may be divided into three separate multiwell plates (e.g. PCR plates comprising 96 wells) to separately amplify IGH, IGK, and IGL, respectively.

The well position of the original single cell is the same for each plate, i.e. "well A1" of each of the IGH, IGK, and IGL plates were amplified from "well A1" of the original plate.

The inventors have developed a set of target primers designed to promote amplification of the immune receptor chain sequences, (either the BCR chain sequences or the TCR chain sequences), which comprise the variable (V), diversity (D), joining (J), and constant (C) regions.

In each well of the multiwell plate(s), the cDNA template is contacted with a polymerase, typically a DNA polymerase, and a set of target primers under conditions which allow for amplification to be accomplished in each well. The set of target primers is typically comprised in a primer mixture, which is added to each well of the multiwell plate(s).

As used herein, the term "multiwell plate" means a plate comprising a plurality of wells, wherein each well serves as an individual reaction vessel. Typically, the multiwell plate is a PCR plate comprising at least 96 wells.

As used herein, the term "set of target primers" means a plurality of reverse target primers and forward target primers. Each of the reverse target primers may contain a reverse target sequence which is specific to that reverse target primer. Likewise, each of the forward target primers may contain a forward target sequence, which is specific to that forward target primer. The set of target primers may target a specific gene segment, gene, a specific category of genes or a plurality of genes and/or gene categories. The set of target primers are designed to amplify the immune receptor chain sequences (either the BCR chain sequences or the TCR chain sequences).

The set of target primers may comprise from 5 to 250, preferably from 15 to 150 different target primers.

The number of target primers in the set of target primers depends on the analysis to be performed. In some scenarios, a smaller number of target primers may be sufficient, whereas a larger number may be used to enable a more full-scale analysis. If a more comprehensive analysis is to be performed, a range of from 15 to 150, preferably from 30 to 150, reverse and forward target primers capture the most important gene segments.

It is worth noting that an individual reverse or forward target primer can target more than one gene segment or gene. Accordingly, the number of individual forward and reverse primers required to target a set of target genes can be less than the number of targets.

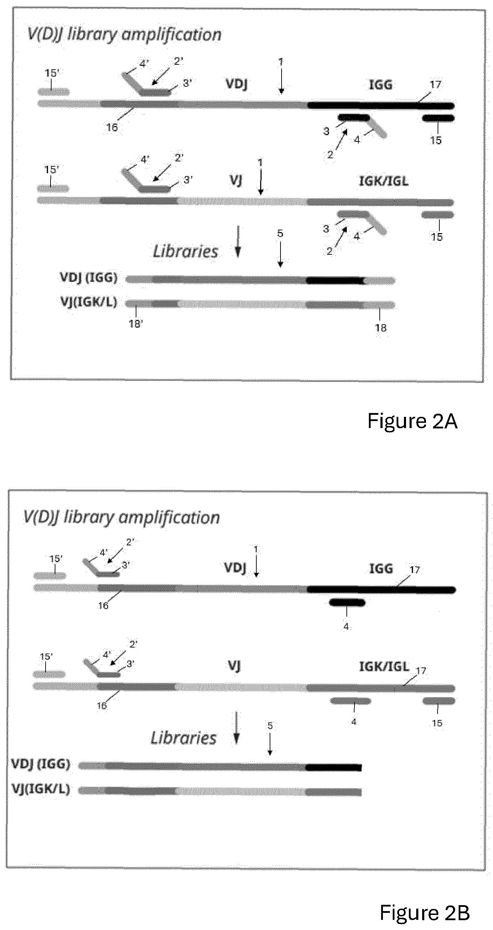

As illustrated in e.g. figures 1 and 2A-B, each of the immune receptor chain sequences 1 comprises
- a variable (V) region, a joining (J) region, and optionally a diversity (D) region;
- a leader region 16 forming the 5' end of each immune receptor chain sequence 1; and
- a constant region 17 forming the 3' end of each of the immune receptor chain sequences 1.

As best illustrated in figure 2A and 2B, the set of target primers comprises a plurality of reverse target primers 2 and forward target primers 2'.

Typically, the reverse target primer 2 targets the constant region 17 of the immune receptor chain sequence 1, and wherein the forward target primer 2' targets the leader region 16 of the immune receptor chain sequence 1.

As used herein, the term "targets" means annealing to a complementary nucleotide sequence under suitable conditions, e.g., as used during polymerase chain reactions (PCR). In the context of primers, "targets" refers to the ability of a primer to hybridize to a designated region of a nucleic acid sequence or an amplicon thereof.

The forward target primer 2 typically comprises the forward bridging sequence 4' and a forward target sequence 3'; the forward target sequence 3' targeting the leader region 16, wherein the forward bridging sequence 4' is arranged 5' of the forward target sequence 3' (see 2A-B).

The reverse target primer 2 comprises either
- the reverse bridging sequence 4, and wherein the reverse bridging sequence 4 targets the constant region 17 (see figure 2B), or
- the reverse bridging sequence 4 and a reverse target sequence 3; the reverse target sequence 3 targeting the constant region 17, wherein the reverse bridging sequence 4 is arranged 5' of the reverse target sequence 3 (see figure 2A).

As used herein, the term "bridging sequence" means a sequence which enables a link or "bridge" to subsequent amplifications. For example, the bridging sequences of the target primers in step b) provide a bridge to amplifications performed in the labelling step d). A bridging sequence may also be referred to as a "universal sequence".

In the embodiment illustrated in figure 2B, the reverse bridging sequence 4 also has a "targeting function" in that it is configured to hybridize to the constant region 17 of the immune receptor chain sequence.

The reverse bridging sequence 4 may be common to all reverse target primers 2 used in the method (which is the case for the embodiment illustrated in figure 2A). The forward bridging sequence 4' may be common to all forward target primers 2' used in the method.

The reverse bridging sequence 4 is typically different from the forward bridging sequence 4'. This allows the subsequent labelling or "bridging" step to be specific to one or the other end.

As mentioned hereinbefore, the bridging sequences of the target primers form "bridges" or links to subsequent amplifications.

As illustrated in figure 1 and in figure 2A, the reverse bridging sequence 4 typically forms the 5' end of the reverse target primer 2. The 5' end of the reverse target primer may also be referred to as the "outer end" of the reverse target primer.

The forward bridging sequence 4' typically forms the 5' end of the forward target primer 2'. The 5' end of the forward target primer may also be referred to as the "outer end" of the forward target primer.

The amplified libraries 5 obtained in each well in step b) will contain a plurality of target regions comprising or corresponding to the constant (C), variable (V), joining (J), and optionally diversity (D) region associated with the respective immune receptor chain derived from the individual cDNA template, i.e. the original single cell.

As used herein, the term "amplified library" means an amplified product comprising a plurality of target regions comprising or corresponding to the constant (C), variable (V), joining (J), and optionally diversity (D) regions. The amplified library 5 may further comprise two bridging regions, i.e. a reverse bridging region (denoted 18 in figure 2A) and a forward bridging region (denoted 18' in figure 2A). As illustrated in figure 2A, the target region is arranged between the reverse 18 and the forward 18' bridging regions.

The bridging regions correspond to or are complementary to the bridging sequences of the target primers.

The amplified library or amplified product may also be referred to as a "multiplexed product" or a "multiplexed library". Multiplexing is a term that refers to multiple samples being processed at the same time. Multiple different DNA sequences are amplified simultaneously in the amplification steps of the method of the present disclosure. Hence, DNA sequences in a plurality of loci in the genomic DNA sample are generated and comprised in the multiplexed product.

The bridging regions (18, 18') form binding ("target") sites for the well-index primers in step d) of the method.

The reverse bridging region in the embodiment illustrated in figure 2B may correspond or be complementary to a region of the constant (C) region of the immune receptor chain sequence.

In step c), the amplified libraries 5 are combined to form a combined set of libraries 5'.

Hence, if e.g. three separate multiwell plates are utilized to amplify the IGH, IGK, and IGL chains, respectively, the "well position A1" of each plate is associated with the same individual cDNA template, i.e. derived from the same single B-cell.

In step c), the "A1 wells" are combined such that a sample comprising a combined library is obtained.

For example, a sample (about 1- 5 µl, e.g. 2-4 µl) may be taken from "well A1" on the "IGH plate", "well A1" on the "IGK plate", and "well A1" of the "IGL plate". These samples may then be combined, typically by transferring the combined samples to an individual well of a multiwell plate.

Accordingly, a combined library 5' will comprise one single heavy chain (IGH), and one single light chain (either IGK or IGL) per well, derived from the heavy and light chains of the original B-cell.

Hence, each well will comprise a combined library comprising a plurality of target regions encompassing the constant (C), variable (V), joining (J), and optionally diversity (D) regions associated with the heavy, and light chain, respectively, derived from a single immune cell.

The method may further comprise a step c') of purifying the combined libraries prior to the labelling step d).

For example, the purification may involve Ampure magnetic beads to remove potential unincorporated primers, and buffer. The purified samples may be eluted from the magnetic beads using e.g. DNAse free H₂0 (30 minutes).

The purified combined library may then be transferred to at least one separate multiwell plate. Typically, a plurality of multiwell plates is utilized.

In step d), the combined libraries 5' are labelled. Each of the combined libraries 5' in the set of combined libraries is labelled with well-specific index sequences (10, 10') and at least one plate-specific index sequence (12,12').

This is accomplished by contacting the combined libraries obtained in step c) with a well-index primer pair (6,6') and a plate-index primer pair (7,7').

This step is typically performed in a plurality of second multiwell plates.

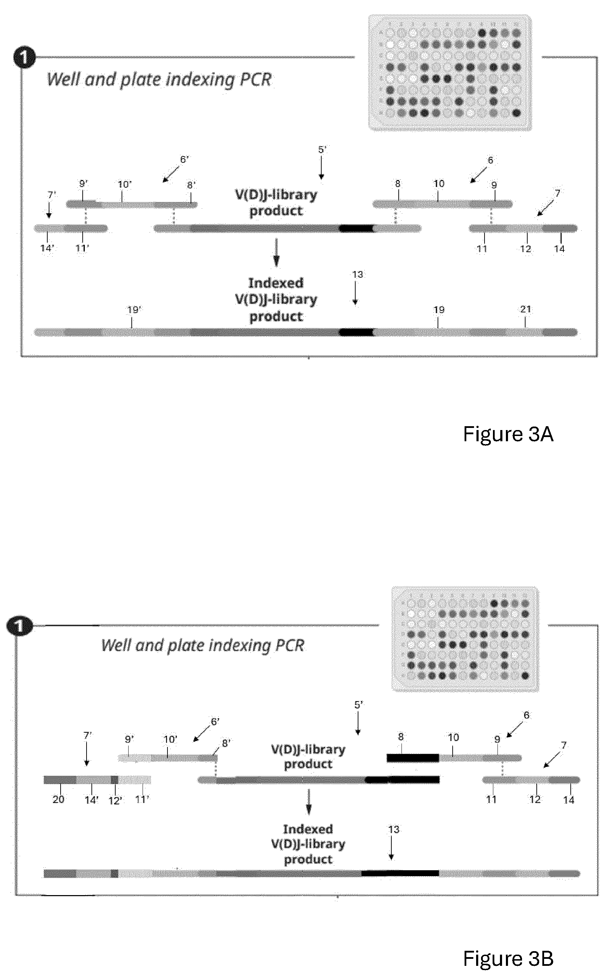

As illustrated in figure 3A, the "well-index primer pair" contains a reverse well-index primer 6 and a forward well-index primer 6'. Each of these well-index primers contains a well-specific sequence (10,10'), a first well-bridging sequence (8,8') and a second well-bridging sequence (9,9').

Different well-index primer pairs (6,6') are used in each well of the plurality of second multiwell plates used in the method.

As illustrated in figure 1, the well-specific sequence (10,10') is arranged between the first well-bridging sequence (8,8') and the second well-bridging sequence (9,9').

The "well-specific index sequence" is a sequence which allows the combined library of a specific well (associated with an individual cDNA template) to be labelled, and to be subsequently extracted from the sequenced library obtained in step f) of the method.

The first reverse well-bridging sequence 8 targets the reverse bridging sequence 4 of the reverse target primer 2. Likewise, the first forward well-bridging sequence 8' targets the forward bridging sequence 4' of the forward target primer 2'.

The second reverse well-bridging sequence 9 forms the 5' end of the reverse well-index primer 6. The 5' end may also be referred to as the "outer end" of the reverse well-index primer 6. The second reverse well-bridging sequence 9 enables subsequent labelling with a reverse plate-specific index 12. Hence, the second reverse well-bridging sequence 9 forms a "bridge" to subsequent "labelling" amplifications.

The second forward well-bridging sequence 9' forms the 5' end of the forward well-index primer 6'. The 5' end may also be referred to as the "outer end" of the forward well-index primer 6'. The second forward well-bridging sequence 9' forms a "bridge" to subsequent amplifications.

The plate-index primer pair (7,7') contains a reverse plate-index primer 7 and a forward plate-index primer 7'. At least one of these plate-index primers contains a plate-specific sequence (12,12'). Furthermore, each of the plate-index primers (7,7') contains at least one first plate-bridging sequence (11,11').

Identical plate-index primer pairs (7,7') are used for each well within each individual second multiwell plate of the plurality of second multiwell plates. In other words, the same plate index primer pair (7,7') is used for each well within each individual second multiwell plate of the plurality of second multiwell plates, but different plate index primer pairs (7,7') are used for different second multiwell plates within said plurality of second multiwell plates.

The "plate-specific index sequence" is a sequence which allows the library of a specific plate, i.e. from a specific second multiwell plate to be extracted from the sequenced library obtained in step f).

As illustrated in figures 3A and 3B, the reverse plate-index primer 7 comprises a first reverse plate-bridging sequence 11. The first reverse plate-bridging sequence 11 is configured to target the second reverse well-bridging sequence 9 of the reverse well-index primer 6.

The forward plate-index primer 7' may comprise at least first forward plate-bridging sequence 11'. The first forward plate-bridging sequence 11' is configured to target the second forward well-bridging sequence 9' of the forward well-index primer 6'.

The reverse plate-index primer 7 may further comprise a second reverse plate-bridging sequence 14. The second reverse plate-bridging sequence 14 may form the 5'end of the reverse plate-index primer 7.

The forward plate-index primer 7' may further comprises a second forward plate-bridging sequence 14'. The second forward plate-bridging sequence 14' may form the 5'end of the forward plate-index primer 7' (see figure 3A).

In figures 3A and 3B, the plate-specific index sequence 12 is arranged between the first reverse plate-bridging sequence 11 and the second reverse plate-bridging sequence 14.

In the context of the present disclosure, it is equally conceivable that the plate-specific index sequence 12' is arranged between the first forward plate-bridging sequence 11' and the second forward plate-bridging sequence 14'.

The first 11 or second 14 reverse plate-bridging sequence and/or the first 11' or second 14' forward plate-bridging sequence may form target or binding sites for sequencing primers used in the subsequent sequencing step.

As mentioned hereinbefore, one or both of the reverse 7 and forward 7' plate-index primers may comprise a plate-specific index sequence (12,12').

In the embodiment illustrated in figure 3A, the reverse plate-index primer 7 comprises a plate-specific index sequence 12.

In the embodiment illustrated in figure 3B, both the reverse 7 and forward 7' plate-index primers comprise a plate-index-specific sequence (12,12').

As illustrated in figure 3B, one of the plate-index primers (7,7') may comprise a third plate-bridging sequence 20. In figure 3B, the forward plate-index primer 7' comprises the third plate-bridging sequence 20, which forms the 5' end of the primer.

The third plate-bridging sequence 20 may be used to enable binding to the sequencing flow cell in Illumina sequencing.

In step d) of the method, a labelled amplified library 13 is obtained in each well.

The term "labelled amplified library" means an amplified product comprising a plurality of target regions comprising or being complementary to the variable (V), joining (J), constant (C), and optionally diversity (D) regions, a reverse well-specific index region 19, a forward well-specific index region 19', and at least one plate-index region 21.

As illustrated in figure 3A, the reverse well-index region 19 of the labelled amplified library 13 corresponds to or is complementary to the reverse well-index sequence 10 of the reverse well-index primer 6.

Likewise, the forward well-index region 19' of the labelled amplified library 13 corresponds to or is complementary to the forward well-index sequence 10' of the forward well-index primer 6'.

The plate-index region 21 corresponds to or is complementary to the plate-index sequence 12 of one of the plate-index primers (7,7').

Typically, the well-index primers (6,6') are rate-limiting and are used in lower concentrations.

For example, the ratio of the well-index primer pairs (6,6') to the plate-index primer pairs (7,7') used in the method may be at least 1:10, preferably from 1:20 to 1:60.

Hence, the labelling procedure, or "bridge indexing procedure", may occur in two stages. Each combined library of step c) functions as the template for the well index-primers (6,6'). As the reaction proceeds, the well-index primers (6,6'), being present at a lower concentration, become depleted. This initial stage results in the addition of the well-specific index sequences (10,10') and the well-bridging sequences (8,8' and 9,9') to the template. This extended template now contains added bridging sequences, that can now be targeted by the plate-index primers (7 7'), such that the plate-specific index sequence(s) (12,12') and plate-bridging sequences (11,11' and/or 14,14') to the reaction product.

The reverse well-index primer 6 may have a sequence as defined in any one of SEQ ID NO:302-397 or a corresponding sequence having at least 95%, preferably at least 99 % sequence homology with any one of SEQ ID NO:302-397.

The forward well-index primer 6' may have a sequence as defined in any one of SEQ ID NO:206-301 or a corresponding sequence having at least 95%, preferably at least 99 % sequence homology with any one of SEQ ID NO:206-301.

The reverse plate-index primer 7 may have a sequence as defined in any one of SEQ ID NO: 182-205 or a corresponding sequence having at least 95%, preferably at least 99 % sequence homology with any one of SEQ ID NO: 182-205.

The forward plate-index primer 7' may have a sequence as defined in SEQ ID NO: 181 or a corresponding sequence having at least 95%, preferably at least 99 % sequence homology with SEQ ID NO:181.

The labelled amplified libraries 13 obtained in step d) may be purified prior to step e).

For example, each of the labelled amplified libraries of the second multiwell plates may be prepared separately by combining equal amounts of each of the labelled amplified libraries and purified in two steps, e.g. by agarose gel purification, followed by magnetic bead purification. One plate-specific library may be mixed with additional plate-specific libraries, each containing different plate-specific indexes but purified using the same steps.

In step e), the labelled amplified libraries 13 of each well of the plurality of second multiwell-plates are combined to yield a combined labelled library.

The "combined labelled" comprises the plurality of labelled libraries obtained in step d). Hence, the combined labelled library comprises a plurality of sub-libraries; i.e. multiple sets of labelled amplified libraries 13, each of which contains a set of target regions associated with the immune receptor chains of a single immune cell. Each set of target regions is associated with the amplification performed in one individual well of the first multiwell-plate.

Since each labelled amplified library 13 is labelled with two well-specific index sequences, and with a plate-specific sequence, an individual sub-library (i.e. an individual amplified library) contained in the combined labelled library may be subsequently analyzed and compared with different sub-libraries and/or with a predefined gene reference set.

Step f) of the method comprises sequencing the combined labelled library to provide a sequenced library.

The present disclosure is not limited to a specific sequencing technique or a specific sequencing instrument. Any sequencing technique or instrument known to the skilled person may be utilized.

The sequencing is preferably performed by next-generation sequencing (NGS). For example, an Illumina MiSeq or Illumina HiSeq instrument may be used.

The multiple amplifications performed in the various steps of the method may be sequenced in one single run.

The sequencing step f) may comprise providing a reverse sequencing primer targeting the reverse plate-index primer 7 or a segment thereof and a forward sequencing primer targeting the forward plate-index primer 7' or a segment thereof. For example, either the first or the second plate-bridging sequences of the plate-index primer pairs (7,7') may be targeted by the sequencing primers.

Step g) of the method comprises analyzing the sequenced library to identify the pairing of immune receptor chain sequences derived from each individual cDNA template. Preferably, this is accomplished by bioinformatic analysis.

Accordingly, step g) may comprise a bioinformatic analysis of the sequenced library generated in step f).

A bioinformatic analysis is beneficial to analyze and interpret large data sets. Hence, the method of the present disclosure enables the identification and determination of paired immune receptor chain sequences in a high-throughput and rapid manner.

The analyzing in step g) may comprise evaluating variable (V), joining (J), diversity (D), and/or constant (C) regions associated with the immune receptor chain sequences to identify receptor clonotype composition, and/or assess sequence diversity.

Step g) may be performed by a processing unit.

The processing unit may in some embodiments form part of a computer system, further comprising a database. The computer system may be implemented as a server. It may be possible to make use of more than a single processing unit, such as for example including FPGAs, ASICs, etc., software systems and applications, software packages, mechanical and electrical parts, etc. Software packages may be part of the server, the database, and/or a network.

Step g) may comprise:
i) extracting, from the sequenced library, the combined amplified libraries 5' belonging to a specific second multi-well plate by using the plate-specific index sequence(s) (12,12');
ii) extracting, from the sequenced library, the combined amplified libraries 5' belonging to a specific well by using the well-specific index sequences (10,10');
iii) evaluating the variable (V), joining (J), diversity (D), and/or constant (C) regions associated with each combined amplified library 5';
iv) comparing the sequences of the variable (V), joining (J), diversity (D), and/or constant (C) regions in each combined amplified library 5' with a reference database to evaluate gene segment usage, identify allelic variants, and/or to assign lineage specific complementary determining region 3 (CDR3) sequences for each combined amplified library 5'; and optionally
v) determining a consensus immunoglobulin heavy chain (IGH) and a consensus light chain, selected from kappa light chain (IGK) and lambda light chain (IGL) for the B-cell receptor chains, and/or a consensus T-cell receptor alpha or gamma chain, and/or a consensus T-cell receptor beta or TCR delta chain for the T-cell receptor chains.

Steps i) and ii) may be referred to as "demultiplexing steps". The sequenced library obtained in the method may also be referred to as a "multiplexed library".

Demultiplexing is the process of separating this multiplexed library into individual amplified libraries (sets of sequences) that correspond to specific single cells. This is achieved by using the unique well- and plate-index sequences that were introduced during the labelling step d). These index sequences act like tags that identify which sequences came from which well and plate, enabling the processing unit to group sequences by their original single cell.

Once demultiplexed, the sequences of the variable (V), joining (J), diversity (D), and/or constant (C) regions in each combined amplified library 5' may be compared with a reference database. This step may be performed by a processing unit.

The processing unit may be configured to compare each sequence to a database of known immune receptor genes, e.g. using tools like IgBlast. This process can identify which specific V (variable), D (diversity), J (joining), and C (constant) gene segments were used to create the receptor chain. It may also identify the complementary determining region 3 (CDR3), which is a critical part of the receptor involved in binding antigens and determines the receptor's specificity.

The method may further comprise a step of eliminating non-functional immune receptor chains.

For example, the processing unit may be configured to exclude such non-functional immune receptor sequences such that only functional immune receptor chains are included in the analysis.

The non-functional immune receptor chains may be chains containing a stop codon or chains failing to maintain the reading frame from the V, D, J, or C regions.

Furthermore, for each single cell, a consensus immunoglobulin heavy chain (IGH) and a consensus light chain, selected from kappa light chain (IGK) and lambda light chain (IGL) for the B-cell receptor chains, and/or a consensus T-cell receptor alpha or gamma chain, and a consensus T-cell receptor beta or TCR delta chain for the T-cell receptor chains, may be determined.

For example, the processing unit may be configured to evaluate all the functional sequences available for the heavy chain and light chain (for immunoglobulins) or alpha/beta chains (for TCRs). The processing unit may be configured to calculate a "consensus sequence," being an average or most representative sequence.

If enough data is available, the processing unit may be configured to generate a single consensus heavy chain and a single consensus light chain for immunoglobulins, or a single alpha and beta chain for TCRs. For the less common TCR types (like gamma/delta chains), the same principle applies.

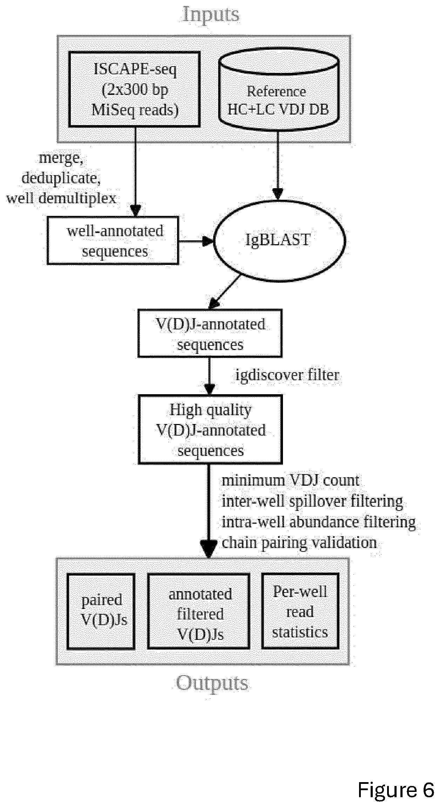

Hence, the method allows for accurate pairing, and that sequences from each single cell are kept together. Figure 6 schematically illustrates an exemplary computational workflow of the method of the present disclosure.

As illustrated in e.g. figure 2A, the reverse 2 and forward 2' target primers of the set of target primers may be inner target primers; the set of target primers further comprising a plurality of outer target primers (15,15'), wherein the polymerase chain reactions (PCRs) in step b) are nested PCR amplifications comprising:
- a first PCR amplification, in which the plurality of outer target primers (15,15') is used to generate a plurality of amplicons, and
- at least a second PCR amplification, in which the inner target primers are used to anneal to sequences within the amplicons generated in the first PCR amplification.

Accordingly, the specificity and sensitivity of the amplification step may be enhanced.

The method may further comprise a step of performing a retailing polymerase chain reaction (PCR) after step f), wherein the retailing PCR utilizes a set of retailing primers configured to amplify and extend the leader region 16 and the constant region 17 (see figure 1, box denoted 2).

The set of retailing primers may comprise a plurality of forward retailing primers and at least one reverse retailing primer.

These retailing primers are used to extend the leader region 16 and the constant region 17 to facilitate cloning into an expression vector.

As mentioned hereinbefore, the leader region 16 is located upstream of the V, D, or J regions of the immune receptor chain. The constant region 17 is located downstream of the V, D, or J regions.

The forward and reverse retailing primers may be defined by any one of the sequences defined in SEQ ID NO:398-480.

The identification of which light chain is used in the amplified library determines which light chain retailing primer set to be used for that well (IGK or IGL).

After the retailing PCR step, the amplicons may be purified using e.g. Ampure magnetic beads.

The retailing PCR may be beneficial to facilitate subsequent cloning.

According to another aspect, there is provided a method for generating monoclonal antibodies or T-cell receptors comprising:
i) identifying paired adaptive immune receptor chain sequences as described hereinbefore;
ii) cloning at least one or a plurality of paired adaptive immune receptor chain sequences identified in step i) into an expression vector; and
iii) expressing the paired immune receptor chain sequences in a host cell to produce monoclonal antibodies or T-cell receptors.

The method enables the functional evaluation and production of specific immune receptor chains. In some instances, it is desired to select and clone specific receptor chain pairs for cloning and expression. For example, if certain receptor chain pairs show promising antigen-binding characteristics or demonstrate desirable functional properties, those pairs may be chosen for cloning and expression. Pairs identified from antigen-specific cells in disease contexts (e.g., autoimmune disorders, cancer, or infectious diseases) may hold therapeutic potential.

Furthermore, the method may be used to study immune receptor diversity and function, which may be important for the generation of therapeutic monoclonal antibodies and TCR-based therapies. By studying the immune receptor repertoires, valuable insights into immune responses to infections, cancer, and autoimmunity, are enabled.

The method may also be used in personalized vaccine and treatment strategies, as mentioned hereinbefore.

As used herein, the term "expression vector" means a DNA construct, such as a plasmid, designed to introduce a specific gene or genes into a host cell to enable transcription and translation into proteins. An expression vector typically comprises regulatory elements such as a promoter, enhancer, and terminator sequences to control gene expression, and optionally selectable markers to facilitate the identification of successfully transformed host cells.

In exemplary embodiments, the expression vector in step ii) is a plasmid configured for expression in mammalian cells.

In exemplary embodiments, the cloning in step (ii) comprises joining the immune receptor chain sequences and the expression vector by a ligase-free process.

As used herein, the term "ligase-free process" means a method of joining DNA fragments that does not require the use of DNA ligase enzymes. Instead, such processes typically utilize homologous recombination or similar mechanisms to assemble DNA fragments. Examples of ligase-free processes include Gibson assembly, which relies on complementary sequences at the ends of DNA fragments to facilitate precise joining.

For example, the cloning in the step (ii) may comprise joining retailing PCR products obtained in the retailing PCR step and the expression vector by a ligase-free process.

The retailing PCR, as described hereinbefore, may prepare the immune receptor chain sequences for efficient incorporation into an expression vector by adding specific 5' and 3' extensions to the sequences. These extensions provide homology to the expression vector and facilitate the integration during the cloning process.

The cloning step (step ii) may utilize these extensions to join the retailing PCR products with the expression vector in a ligase-free process, such as Gibson assembly. This process aligns complementary regions on the PCR products and vector, enabling homologous recombination without the need for enzymatic ligation.

Extensions to facilitate ligase-free cloning may also be accomplished in alternative ways.

For example, the second forward well-bridging sequence 11' (see figure 3B) may also be an expression vector overlap sequence. As mentioned hereinbefore, the reverse bridging sequence 4 of the reverse target primer 2 may correspond or be complementary to the constant region of the immune receptor chain sequence. This configuration enables in-frame cloning of the "retailed" recombinant sequence into the appropriate expression vector.

### EXAMPLES

### Methods

### Molecular pipeline

Single B cell cDNA templates were first generated by reverse transcription of RNA extracted from lysed cells in the 96 well plates using the reverse transcriptase SuperScriptTM IV (Invitrogen), with a mix of oligo-dT and random hexamer primers. This cDNA forms the template for the subsequent amplification of the heavy and light chain recombinants.

A two round 96 well plate-based amplification of V(D)J regions was performed by three separate 20 µl multiplex nested PCR reactions for IGG (heavy chain), and IGK and IGL (light chains) in KAPA HiFi HotStart ReadyMix (Roche Molecular Systems) from 6 µl of HC and 3 µl of KC or LC cDNA template. 1 µl of the first-round reaction from each well served as a template for the nested reaction. Respective 5' leader IGHV/IGKV/IGLV and 3' constant IgG/IgK/IgL primer mixtures were used at 10 pmol concentration for the first and second PCR round. Following PCR amplification, 3 µl of each of the three separate reactions are combined and purified using AMPure XP magnetic beads (Beckman Coulter) to remove PCR primers and buffer. The purified inner PCR amplicons are eluted from the magnetic beads using 35 µl of DNAse-free H2O and comprise a paired heavy chain (IgG) and light chain (either IgK or IgL) per well of the original single B cell. This purified product is subsequently utilized for NGS analysis, following indexing, or for cloning into expression vectors, following the leader/constant retailing procedure. All primer sequences used in the Examples are defined by SEQ ID NO:1-457 in the sequence list.
SEQ ID NO: 1-2 define the sequences of the primers used for cDNA synthesis.
SEQ ID NO:3-37 define the sequences of the IGHV outer forward target primers used.
SEQ ID NO:38-43 define the sequences of the IGG outer constant target primers used.
SEQ ID NO:44-69 define the sequences of the IGHV inner forward target primers used.
SEQ ID NO:70 defines the sequences of the IGG inner constant primer used (second round IGG).
SEQ ID NO:71-86 define the sequences of the IGK outer forward primers used.
SEQ ID NO:87 defines the sequence of the IGK outer constant primer used (first round IGK).
SEQ ID NO:88-108 define the sequences of the IGK inner forward primers used.
SEQ ID NO 109 defines the sequence of the IGK inner constant primer used (second round IGK).
SEQ ID NO:110-141 define the sequences of the IGL outer forward primers used.
SEQ ID NO:142 defines the sequence of the IGL outer constant primer used (first round IGL).
SEQ ID NO:143-175 define the sequences of the IGL inner forward primers used.
SEQ ID NO:176 defines the sequence of the IGL inner constant primer used (second round IGL).
SEQ ID NO:177 defines the sequence of the control outer forward primer used.
SEQ ID NO:178 defines the sequence of the control outer reverse primer used.
SEQ ID NO:179 defines the sequence of the control inner forward primer used.
SEQ ID NO:180 defines the sequence of the control inner reverse primer used.
SEQ ID NO:181 defines the sequences of the forward plate-index primer used.
SEQ ID NO:182-205 define the sequences of the reverse plate-index primers used.
SEQ ID NO 206-301 define the sequences of the forward well-index primers used.
SEQ ID NO 302-397 define the sequences of the reverse well-index primers used.
SEQ ID NO:398-424 define the sequences of the IGHV retail forward primers used.
SEQ ID NO:425 defines the sequence of the IGG retail constant primer used.
SEQ ID NO:426-446 define the sequences of the IGKV retail forward primers used.
SEQ ID NO:447 defines the sequence of the IGK retail constant primer used.
SEQ ID NO:448-479 define the sequences of the IGLV retail forward primers used.
SEQ ID NO:481 defines the sequence of the IGL retail constant primer used.

### Bridge indexing and next generation sequencing (NGS)

The inner IGG, IGK and IGL primers contain a common 5' primer forward tail ("forward bridging sequence") and a common 5' reverse primer tail ("reverse bridging sequence"), enabling a combined well and plate indexing step using a bridge PCR procedure. Four microliters of the purified combined template is indexed using the well- and plate-index primer mix (comprising the appropriate well and plate index primers defined in SEQ ID NO: 181-397). The forward and reverse well index primers are rate limiting and are used at 0.25 pmol, while the outer plate-index primers are used at 10 pmol.

The well-index primers are incorporated in the first few rounds of the index PCR reaction, and subsequently the outer plate-index primers taking over during the later index rounds, resulting in a full-length amplicon containing both well- and plate-indexes after 11 PCR cycles. The amplified indexed products from a single plate are combined, purified using magnetic beads such as Ampure beads, and adjusted to the appropriate concentration for subsequent sequence analysis of each plate library using the Illumina V3 2 x 300 cycle kit. The identification of which light chain is utilized determines which of the light chain retailing primer sets, IGK or IGL, to use for that well.

### Retailing and mAb cloning

To clone the heavy and light chain amplicons into an expression vector capable of producing functional full length heavy and light chains, the universal forward and reverse tails may be replaced with the appropriate full-length leader and constant regions sequences. Three multiplex set of primers were designed that can bind to the original primer target sequences in the inner PCR library, enabling the full-length extension of the leader of any expressed heavy, Kappa and Lambda genes present. The full-length leader primers also contain a cloning overlap of 20 bp that facilitates ligation-free cloning. The leader multiplex primer sets are used in conjunction with the appropriate IGG, IGK or IGL constant extension primers. The retailing primers used correspond to the sequences defined in SEQ ID NO:398-480. The retailing primers for IGG chains are defined in SEQ ID 398-425. The retailing primers for the IGK chains are defined in SEQ ID 426-447, and the retailing primers for the IGL chains are defined in SEQ ID 448-480.

PCR retailing with a 10 pmol multiplexed set of upstream gene-specific leader primers and downstream vector overlap primers replicated functional and in-frame insert overlaps for subsequent target-specific mAb cloning. 4 µl of the purified inner PCR product mix served as template for separate IGG and IGK or IGL reactions. Retailed purified PCR-products containing the leader sequence and HC VDJ and LC VJ sequences from antigen-specific single B cells were directly (ligation-free) cloned into expression vectors (Addgene) upstream of the human IgG1, IgK1 or IgL2 constant regions. HC VDJ sequences of LPAF-a class variants were germline reverted while preserving the mature HCDR3 sequence, synthetized with L52 or R52 and overhangs matching the expression vector, and processed likewise. In brief, Gibson assembly reactions with 50 ng of restriction enzyme-digested vector, 30 ng of corresponding overlap insert and 10 µl of Gibson master mix (New England Biolabs) were incubated at 50°C for 1 hour before transformation into XL10-Gold ultracompetent cells (Agilent Technologies) by heat shock at 42 °C for 45 seconds. Colonies were PCR screened, Sanger sequenced (Genewiz) and sequence results were aligned with the original V(D)J insert sequence to identify positive colonies containing 100% identical plasmids. Consecutively, positive colonies were expanded, and plasmids purified with the Plasmid Plus Midi Kit obtained from Qiagen.

### Results

### Study design and the "ISCAPE technique"

The high-throughput single cell antibody sequencing and monoclonal antibody isolation technique of the present disclosure, referred to herein as "ISCAPE" is shown in **figure 1****.** The approach couples personalized genotyping to define the precise IG germline alleles present in each study participant with sorting of single antigen-specific B cells into 96 well plates for paired heavy and light chain sequencing. Specifically, the "ISCAPE technique" comprises the following steps. First RNA from single sorted cells serves as a template for cDNA synthesis using a mixture of random hexamer and oligo dT primers. The cDNA template produced enables nested multiplex PCR for the HC and LC (IGK and IGL), performed in three separate 96-well plates. Amplification of HC VDJ regions is achieved using the 5' IGHV outer forward primers and the IGG outer constant primers. Amplification of light chain VJ regions is achieved using the 5' IGKV outer forward primers and the 5' IGLV outer forward primers with the IGK or IGL 3' outer primers each, respectively. Using the product as a template, nested PCR of the HC VDJ sequence is performed by using the 5' IGHV inner forward primers and the IGG inner constant primers. Nested PCR of the light chain VJ sequences is subsequently performed using the 5' IGKV inner forward primers and the IGLV 5' inner forward primers with the IGK or IGL 3' inner constant primers each.

The product from the nested PCR may be used for two purposes: 1) a combined well and plate indexing PCR step that enables high throughput sequencing of multiple sorted plates and 2) a retailing PCR to generate inserts that are suitable for cloning the HC/LC sequences into expression vectors for transfection of HEK 293F cells and purification of IgG for functional and structural studies **(****Figure 1****).**

The well and plate indexing step makes use of the universal forward (UF) tail ("forward bridging sequence") and the reverse universal (UR) tail ("reverse bridging sequence") introduced by inner PCR for each amplicon (IGG, IGK and IGL), adding a well and plate index to each HC and LC pair using a bridge PCR procedure.

Following next generation sequencing, the combination of well and plate indexes enables the computational disambiguation of well specific libraries from multiple combined plates using the "ISCAPE" script, thereby identifying a single HC VDJ sequence and a single light chain VJ sequence, either IGK or IGL, per well. In brief, the computational pipeline identifies paired receptor chains while controlling for technical artifacts and contamination. Paired-end sequencing reads are merged, deduplicated, and demultiplexed based on dual index combinations corresponding to individual plate and wells, and aligned to reference V, D, and J gene segments using IgBLAST (see **figure 6**). For sequences selected for expression, the purified PCR amplicons may be processed for insertion into expression vectors using a retailing technique that restores the full-length leader for each HC and LC sequence. The PCR retailing removes the index associated UF and UR tails and extends the full-length 5' leader sequence of each antibody sequence, thereby enabling directional cloning into an appropriate IGG, IGK or IGL expression vector. The retailed sequences maintain the functional reading frame from the beginning of the leader, through the VDJ or VJ sequence and into the constant region. The 5' retailing forward primers comprise a multiplex set for IGG, IGK and IGL and extend each gene-specific leader to result in a full leader sequence containing the initiation ATG methionine codon plus an additional 5' tail that overlaps the expression vector promoter region. The 3' retailing primer used in the case of IGG, IGK or IGL binds to the section of the constant region present in the inner PCR reaction and extends it further into the appropriate constant region. The retailing primers used in the method are defined by SEQ ID NO:398-480. This results in the production of in-frame amplicons that are suitable for ligation-free cloning into HC- or LC-specific expression vectors using Gibson cloning. The retailing step is typically performed on all wells in the plate such that any HC/LC pairs could be taken forward for cloning and expression of mAbs.

As mentioned hereinbefore, there are several applications of the method of the present disclosure ("the ISCAPE technique"), including multi-donor analyses to identify differences and commonalities in antigen-specific responses between individuals and populations. The method can also be used to study intra-individual antibody composition and lineage evolution, and it allows rapid mAb isolation. Finally, the method of the present disclosure generates high quality data of paired HC/LC sequences from antigen-sorted cells that can be used for machine learning approaches to better predict antibody-antigen interactions **(****Figure 1****).**

### The HA-specific memory B cell repertoire is highly personal

In the present study, we identified three volunteers, F1, F21 and F22, with a history in Influenza A exposure who displayed robust memory B cell populations following staining with biotinylated H1N1_{pdm2009}-based probes. We genotyped each study participant for their IGH, IGK and IGL allele content using IgDiscover and Corecount [1, 2]. For antigen-specific B cell sorting, we used three HA probes: a monomeric HA head protein, a trimeric stem domain and a full-length HA trimer comprising both the head and stem domains [3]. As expected, B cells recognizing the HA head dominated the response compared to HA stem binders **(****Figure 4A****).**

Following library preparation, we sequenced the combined libraries on the Illumina MiSeq instrument using the 2 x 300 bp sequencing kits and analyzed the output with the software developed for the inventive method ("ISCAPE analysis software") **(****Figure 6****).** This yielded 780 full-length productive HC and LC pairs, with 659 pairs from HA trimer probe-binding B cells; 96 pairs from participant F1, 393 pairs from F21 and 170 pairs from participant F22 (**Figure 4B**) for which the mean level of somatic hypermutation (SHM) in the LC and HC was comparable between the cases **(****Figure 4C****).**

To visualize the genetic diversity and SHM of the antibodies, trimer-binding HC and LC pairs were plotted based on their respective IGHV and IGKV/IGLV germline allele usage **(****Figure 4D****).** Clonotypes were grouped by single-linkage clustering based on identical V and J allele assignments and CDR3 length with 80% CDR3 sequence homology. The frequency of each V pairing was calculated in percentages and encoded by color transparency, while the circle size reflects the extent of clonal expansion.

Participant F1 showed the greatest diversity in HC/LC pairings, while F21 was the donor who contributed the most pairs. IGHV and IGKV/IGLV allele usage patterns partially overlapped between F21 and F22, while in F1, overlap with the other participants was limited. Preferred HC/LC V allele pairs that were observed in at least two study participants included IGHV3-9*01 and IGKV1-33*01 (F1, F22), IGHV2-70*01/*01_S0531 and IGLV3-9*01 (F21, F22), IGHV4-39*01/*07 and IGLV2-14*01 (F22, F21) and IGHV3-7*01/*03 and IGKV3-15*01/3D-15*01 (F21, F1, F22). The respective HA trimer-specific HC and LC V allele combinations were also linked to bulk repertoire frequencies and visualized as chord diagrams **(****Figure 4E****).**

Total allele expression data was obtained from bulk sequencing of each donor's IgM and IGK/L repertoire. Comparative over- or underrepresentation in the HA trimer B memory cell pool is encoded by bar height and orientation in the outer circles, for each allele, respectively. Sector size and connecting arcs indicate allele usage and pairing prevalence. Single V alleles that were overrepresented in the HA-specific response compared to the total IGM or IGK/L repertoire of each individual, and that were observed in more than one donor, included IGHV3-9*01 (F1, F22, F21), IGHV2-70*01/*01_S0531 (F21, F22), IGHV4-39*01/*07 (F22, F21), IGLV3-21*02 (F1, F22), IGLV3-9*01 (F21, F22), IGKV3-15*01/3D-15*01 (F1, F21) **(****Figure 4E****).**

### Biased germline allele usage in a public multi-donor class of HA head-targeting antibodies

To examine if any of the HA-binding sequences identified with the method of the present disclosure in our study participants resembled previously described influenza HA antibodies, we used a recently published dataset comprising over 5,500 HA-specific antibodies [4]. This resource summarizes efforts to isolate mAbs against influenza virus HA during the past two decades. Using all antibodies in the "Wang et al. database" [4] from which nucleotide sequences were available and the 659 HA-binding antibody sequences obtained in the current study, we calculated length-normalized edit distances (Levenshtein distance by pairwise string alignment of VH amino acid sequences) and grouped them by hierarchical clustering.

The results were displayed for sequences with < 12.5% nucleotide heterology as a heatmap linked to the combined HC-LC pairing plot from the three donors **(****Figure 5A****).** Several clusters with high sequence homology were identified, one of them comprising 37 antibodies of 15 lineages using IGHV2-70 paired with IGLV3-9. These sequences were isolated in study participants F21 and F22, and feature a stereotypical genetic signature, that was previously described for a multi-donor HA head-binding class of antibodies, the LPAF-a class [5]. This class of antibodies is characterized to be strain-specific, with a high affinity for H1N1 A/California/04/2009 (H1 CA09), which emerged during the 2009 swine flu pandemic. Moreover, currently circulating seasonal H1N1 flu viruses and strains in annual flu vaccines derive from H1 CA09.

The LPAF-a signature was described to comprise the IGHV2-70*01 germline encoded motif Trp₅₃-Asp₅₄ in the HCDR2 and a conserved HCDR3 Tyr-Gly-Asp (Y₉₇G₉₈D₉₉) motif that binds the receptor binding site of HA and accounts for 37.9% of buried surface area (BSA) based on previous structural analyses [5]. This motif is fully germline encoded by IGHD4-17*01. Consistent with this, IGHV2-70-using antibodies from participants F21 and F22 almost exclusively used IGHD4-17*01 with HCDR3 lengths that were very similar to those reported for LPAF class antibodies in Cheung et al., 2020 [5].

As shown by the individualized IG genotyping, we noted that F22 was homozygous for IGHV2-70*01, while F21 was heterozygous for a novel IGHV2-70*01 allele that had the same coding sequence as IGHV2-70*01, IGHV2-70*01_S0531, and for IGHV2-70*15, which encodes a germline-encoded polymorphism in position 50 upstream of the HCDR2 (Kabat numbering scheme): a leucine (L) in IGHV2-70*01, IGHV2-70*01_S0531 and an arginine (R) in IGHV2-70*15 **(****Figure 5B****).** We found that both the IGHV2-70*01_S0531 and IGHV2-70*15 alleles were used in the total IgM repertoire of F21 with comparatively low overall expression of both alleles (IGHV2-70*01_S0531: 0.216%; IGHV2-70*15: 0.529%). The HA-specific response in this individual was almost exclusively restricted to IGHV2-70*01_S0531 **(****Figure 5C****).**

To model if the R in IGHV2-70*15 might be incompatible with elicitation of the LPAF-a class of neutralizing antibodies, we used the structure of antibody LPAF-a.01 in complex with the HA head domain of H1 CA09 (PDB ID 6URM). Rather than directly contacting HA, HCDR2 position 50 is located outside of the LPAF-a antibody paratope, in the interface between the heavy and light chains **(****Figure 5D****).** The modelling suggested that the presence of a hydrophilic amino acid residue (R50) would significantly decrease the hydrophobicity of the heavy chain surface at the HC/LC interface. As HC/LC interfaces in human antibodies favor hydrophobic patches to enable strong pairing between chains [6], R50 may disrupt the hydrophobic interface, destabilizing binding. Additionally, as position 50 is nearby important paratopic residues, a large change in amino acid character between L and R may indirectly destabilize the interaction between the antibody and HA. To investigate this experimentally, we selected two HC/LC pairs of the LPAF-a class from our dataset, HT24 and HT25, and generated versions of both the mature and germline-reverted HCs with and without an L/R change in HCDR2 position 50. For all variants we kept the mature HCDR3s intact, and we transfected each HC with the mature LC of the respective antibody for mAb production. To assess if the polymorphism influenced the function of the mAbs, we tested each variant mAb for its virus neutralizing activity against the H1N1A/Idahoe07/2018 and the H1N1 A/California07/2009 influenza strains. We found that the L/R change reduced the activity of both the mature and germline-reverted mAbs. This was especially apparent when testing neutralizing activity against H1N1 A/California07/2009, which was potently neutralized by both the germline and mature versions of mAbs HT24 and HT25 containing an L in position 50, but not in the presence of an R **(****Figure 5E****).**

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

The control functionality of the present disclosure may be implemented using existing computer processors, or by a special purpose computer processor for an appropriate system, incorporated for this or another purpose, or by a hardwired system. Embodiments within the scope of the present disclosure include program products comprising machine-readable media for carrying or having machine-executable instructions or data structures stored thereon. Such machine-readable media can be any available media that can be accessed by a general purpose or special purpose computer or other machine with a processor. By way of example, such machine-readable media can comprise RAM, ROM, EPROM, EEPROM, CD-ROM or other optical disk storage, magnetic disk storage or other magnetic storage devices, or any other medium which can be used to carry or store desired program code in the form of machine-executable instructions or data structures and which can be accessed by a general purpose or special purpose computer or other machine with a processor. When information is transferred or provided over a network or another communications connection (either hardwired, wireless, or a combination of hardwired or wireless) to a machine, the machine properly views the connection as a machine-readable medium. Thus, any such connection is properly termed a machine-readable medium. Combinations of the above are also included within the scope of machine-readable media. Machine-executable instructions include, for example, instructions and data which cause a general-purpose computer, special purpose computer, or special purpose processing machines to perform a certain function or group of functions. Also, two or more steps may be performed concurrently or with partial concurrence. Such variation will depend on the software and hardware systems chosen and on designer choice. All such variations are within the scope of the disclosure. Likewise, software implementations could be accomplished with standard programming techniques with rule-based logic and other logic to accomplish the various connection steps, processing steps, comparison steps and decision steps.

### References

*[1] Corcoran et al.,* 2016: https://pubmed.ncbi.nlm.nih.gov/27995928/
*[2] Narang et al.,* 2023: https://pubmed.ncbi.nlm.nih.gov/37114042/
*[3] Artse et al.,* 2022: https://pubmed.ncbi.nlm.nih.gov/36405682/
*[4] Wang et al.,* 2024: https://pubmed.ncbi.nlm.nih.gov/39163866/
*[5] Cheung et al.,* 2020: https://pubmed.ncbi.nlm.nih.gov/32877670/
*[6] Chiu et al,* 2019 https://doi.org/10.3390/antib8040055

## Claims

1. A method for identifying paired immune receptor chain sequences comprising:
a) providing a plurality of individual cDNA templates, wherein said individual cDNA templates are synthesized from RNA extracted from single immune cells; said immune cells being B-cells or T-cells;
b) performing polymerase chain reactions (PCRs) on said individual cDNA templates to separately amplify immune receptor chain sequences (1) selected from:
i) immunoglobulin heavy chain (IGH), kappa light chain (IGK), and lambda light chain (IGL) if said immune cells are B-cells; and
ii) T-cell receptor alpha chain (TCRα), T-cell receptor beta chain (TCRβ), T-cell receptor gamma chain (TCRγ), and T-cell receptor delta chain (TCRδ) if said immune cells are T-cells, wherein
said step b) comprises subjecting said individual cDNA templates to a set of target primers targeting said immune receptor chain sequences (1), wherein said set of target primers comprises a plurality of reverse target primers (2) and forward target primers (2'); each of said reverse target primers (2) comprising a reverse bridging sequence (4), and each of said forward target primers (2') comprising a forward bridging sequence (4'), wherein
said step b) is performed in separate wells of at least one first multiwell plate, and wherein an amplified library (5) is obtained in each well;
c) combining the amplified libraries (5) associated with the same individual cDNA template such that a set of combined libraries (5') is provided;
d) labelling each combined library (5') in said set of combined libraries by performing individual labelling amplification reactions in separate wells of a plurality of second multiwell plates by contacting each combined library (5') with a well-index primer pair (6,6') and a plate-index primer pair (7,7');
said well-index primer pair (6,6') comprising a reverse well-index primer (6) and a forward well-index primer (6');
said reverse well-index primer (6) comprising a first reverse well-bridging sequence (8) targeting said reverse bridging sequence (4), a second reverse well-bridging sequence (9), and a reverse well-specific index sequence (10) arranged between said first (8) and second (9) reverse well-bridging sequences;
said forward well-index primer (6') comprising a first forward well-bridging sequence (8') targeting said forward bridging sequence (4'), a second forward well-bridging sequence (9'), and a forward well-specific index sequence (10') arranged between said first (8') and second (9') forward well-bridging sequences, wherein
said plate-index primer pair (7,7') comprises a reverse plate-index primer (7) and a forward plate-index primer (7');
said reverse plate-index primer (7) comprising at least a first reverse plate-bridging sequence (11) targeting said second reverse well-bridging sequence (9);
said forward plate-index primer (7') comprising at least a first forward plate-bridging sequence (11') targeting said second forward well-bridging sequence (9'), wherein at least one of said reverse (7) and forward (7') plate-index primers comprises a plate-specific index sequence (12, 12')) arranged 5' of said first reverse plate-bridging sequence (11) or said first forward plate-bridging sequence (11'), wherein
different well-index primer pairs (6,6') are used for each well within said plurality of second multiwell plates, and wherein identical plate-index primer pairs (7,7') are used for each well within each individual second multiwell plate of said plurality of second multiwell plates,
whereby a labelled amplified library (13) is obtained in each well of said plurality of second multiwell-plates;
e) combining the labelled amplified libraries (13) of each well of said plurality of second multiwell-plates to yield a combined labelled library;
f) sequencing said combined labelled library to provide a sequenced library;
g) analyzing said sequenced library to identify the pairing of immune receptor chain sequences derived from each individual cDNA template.

2. The method according to claim 1, wherein each of said immune receptor chain sequences (1) comprises
- a variable (V) region, a joining (J) region, and optionally a diversity (D) region;
- a leader region (16) forming the 5' end of each of said immune receptor chain sequences (1); and
- a constant region (17) forming the 3' end of each of said immune receptor chain sequences (1).

3. The method according to claim 1 or claim 2, wherein said reverse target primer (2) targets said constant region (17) of said immune receptor chain sequence (1), and wherein said forward target primer (2') targets said leader region (16) of said immune receptor chain sequence (1).

4. The method according to claim 2 or claim 3, wherein said forward target primer (2) comprises said forward bridging sequence (4') and a forward target sequence (3'); said forward target sequence (3') targeting said leader region (16), wherein said forward bridging sequence (4') is arranged 5' of said forward target sequence (3'), and wherein said reverse target primer (2) comprises
- said reverse bridging sequence (4), and wherein said reverse bridging sequence (4) targets said constant region (17); or
- said reverse bridging sequence (4) and a reverse target sequence (3); said reverse target sequence (3) targeting said constant region (17), wherein said reverse bridging sequence (4) is arranged 5' of said reverse target sequence (3).

5. The method according to any one of the preceding claims, wherein said sequencing in said step f) is performed using next-generation sequencing (NGS).

6. The method according to any one of the preceding claims, wherein said analyzing in step g) comprises evaluating the variable (V), joining (J), diversity (D), and/or constant (C) regions associated with said immune receptor chain sequences to identify receptor clonotype composition, and/or assess sequence diversity.

7. The method according to any one of the preceding claims, wherein step g) comprises a bioinformatic analysis of the sequenced library generated in step f).

8. The method according to any one of the preceding claims, wherein step g) comprises:
i) extracting, from said sequenced library, the combined amplified libraries (5') belonging to a specific second multi-well plate by using the plate-specific index sequence(s) (12, 12');
ii) extracting, from said sequenced library, the combined amplified libraries (5') belonging to a specific well by using said well-specific index sequences (10,10');
ii) evaluating the variable (V), joining (J), diversity (D), and/or constant (C) regions associated with each combined amplified library (5');
iv) comparing the sequences of the variable (V), joining (J), diversity (D), and/or constant (C) regions in each combined amplified library (5') with a reference database to evaluate gene segment usage, identify allelic variants, and/or to assign lineage specific complementary determining region 3 (CDR3) sequences for each combined amplified library (5'); and optionally
v) determining a consensus immunoglobulin heavy chain (IGH) and a consensus light chain selected from kappa light chain (IGK) and lambda light chain (IGL) for said B-cell receptor chains, and/or a consensus T-cell receptor alpha or gamma chain, and a consensus T-cell receptor beta or TCR delta chain for said T-cell receptor chains.

9. The method according to any one of the preceding claims, wherein said step g) is performed by a processing unit.

10. The method according to any one of the preceding claims, wherein said reverse (2) and forward (2') target primers of said set of target primers are inner target primers; said set of target primers further comprising a plurality of outer target primers (15,15'), wherein said polymerase chain reactions (PCRs) in said step b) are nested PCR amplifications comprising:
- a first PCR amplification, in which said plurality of outer target primers (15,15') is used to generate a plurality of amplicons, and
- at least a second PCR amplification, in which said inner target primers are used to anneal to sequences within said amplicons generated in said first PCR amplification.

11. The method according to any one of the preceding claims, wherein the ratio of said well-index primer pairs (6,6') to said plate-index primer pairs (7,7') used in said method is at least 1:10, preferably from 1:20 to 1:60.

12. The method according to any one of the preceding claims, wherein said single immune cells are single B-cells, and wherein said set of target primers is configured to target:
- the IGHV genes, and comprises the forward target primers defined by SEQ ID NO:3-37 and/or SEQ ID NO:44-69 or corresponding sequences having at least 95 %, preferably at least 99% sequence homology with SEQ ID NO:3-37 and/or SEQ ID NO:44-69, and at least one of the reverse target primers defined by SEQ ID NO:38-43, and/or SEQ ID NO:70 or corresponding sequences having at least 95 %, preferably at least 99% sequence homology with SEQ ID NO:38-43, and/or SEQ ID NO:70, and/or
- the IGK genes, and comprises the forward target primers defined by SEQ ID NO:71-86, and/or SEQ ID NO:88-108, or corresponding sequences having at least 95%, preferably at least 99 % sequence homology with SEQ ID NO:71-86, and/or SEQ ID NO:88-108, and the reverse target primers defined by SEQ ID NO:87, and/or SEQ ID NO:109, or corresponding sequences having at least 95%, preferably at least 99 % sequence homology with SEQ ID NO:87, and/or SEQ ID NO:109, and/or
- the IGL genes, and comprises the forward target primers defined by SEQ ID NO:110-141, and/or SEQ ID NO:143-175 or corresponding sequences having at least 95%, preferably at least 99 % sequence homology with SEQ ID NO:110-141, and/or SEQ ID NO:143-175, and the reverse target primers defined by SEQ ID NO:142, and/or SEQ ID NO:176 or corresponding sequences having at least 95%, preferably at least 99 % sequence homology with SEQ ID NO:142 and/or SEQ ID NO:176.

13. The method according to any one of claims 2-12, wherein said method further comprises a step of performing a retailing polymerase chain reaction (PCR) after step b), wherein said retailing PCR utilizes a set of retailing primers configured to amplify and extend said leader region (16) and said constant region (17).

14. A method for generating monoclonal antibodies or T-cell receptors comprising:
i) identifying paired adaptive immune receptor chain sequences as defined in any one of claims 1-13;
ii) cloning at least one or a plurality of paired adaptive immune receptor chain sequences identified in step i) into an expression vector; and
iii) expressing said paired immune receptor chain sequences in a host cell to produce monoclonal antibodies or T-cell receptors.

15. The method according to claim 14, wherein said cloning in said step (ii) comprises joining said immune receptor chain sequences and said expression vector by a ligase-free process.
